(19) Europäisches Patentamt
European Patent Office
Office européen des brevets

(11) EP 0 842 965 B1

(12) **FASCICULE DE BREVET EUROPEEN**

(45) Date de publication et mention de la délivrance du brevet:
**03.01.2001 Bulletin 2001/01**

(51) Int Cl.7: **C08G 77/38**, C08G 77/34

(21) Numéro de dépôt: **97402346.7**

(22) Date de dépôt: **06.10.1997**

# (54) Utilisation d'une silicone filtre pour la protection des fibres kératiniques colorées artificiellement contre les effets des radiations UV

Verwendung von ein Silikonfiltersubstanz zum Schutz der Farbe bei keratinisch gefärbten Fasern

Use of a filter silicone for the colour protection of dyed keratinic fibres against the influence of UV-rays

(84) Etats contractants désignés:
**AT BE CH DE DK ES FI FR GB GR IE IT LI LU MC NL PT SE**

(30) Priorité: **15.10.1996 FR 9612564**

(43) Date de publication de la demande:
**20.05.1998 Bulletin 1998/21**

(73) Titulaire: **L'OREAL**
**75008 Paris (FR)**

(72) Inventeurs:
- **Richard, Hervé**
  **93420 Villepinte (FR)**
- **Lagrange, Alain**
  **77700 Coupvray (FR)**
- **Dubief, Claude**
  **78150 Le Chesnay (FR)**
- **Braida-Valerio, Damarys**
  **75012 Paris (FR)**

(74) Mandataire: **Miszputen, Laurent**
**L'OREAL-DPI**
**6 rue Bertrand Sincholle**
**92585 Clichy Cédex (FR)**

(56) Documents cités:
**EP-A- 0 335 777**
**EP-A- 0 383 655**
**EP-A- 0 389 337**
**EP-A- 0 708 108**
**EP-A- 0 709 080**
**EP-A- 0 711 779**
**EP-A- 0 742 003**
**WO-A-92/20690**
**FR-A- 2 642 967**
**FR-A- 2 642 968**
**FR-A- 2 657 351**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 842 965 B1

Printed by Jouve, 75001 PARIS (FR)

## Description

La présente demande concerne l'utilisation d'un organosiloxane porteur d'au moins un groupe absorbant les ultraviolets du type benzotriazole ou du type benzalmalonate pour la préparation d'une composition cosmétique ou dermatologique comme agent de protection de la couleur des fibres kératiniques colorées artificiellement contre les effets des radiations UV en particulier du rayonnement solaire ; lesdites fibres étant teintes par un procédé de teinture par oxydation.

On sait depuis longtemps que notamment la lumière et les agents de lavage ont tendance à agresser la couleur artificielle des cheveux teints. La couleur des cheveux ainsi obtenue après un traitement de teinture s'affaiblit peu à peu ou vire vers des nuances peu esthétiques ou indésirables.

Il est donc apparu nécessaire de protéger la couleur des fibres kératiniques ayant subi un traitement de teinture et en particulier la couleur des cheveux, et de réduire sensiblement les dégradations liées à l'action des agents extérieurs, notamment les radiations UV et plus particulièrement le rayonnement solaire.

La Demanderesse a découvert, ce qui fait l'objet de l'invention, que des silicones filtres portant des groupements absorbant les ultraviolets du type benzotriazole ou du type benzalmalonate, préservaient, de façon surprenante, la coloration des fibres kératiniques teintes artificiellement par un procédé de teinture par oxydation et en particulier des cheveux, en réduisant sensiblement les dégradations liées à l'action des rayonnements UV, en particulier l'action du rayonnement solaire.

L'invention a donc pour objet l'utilisation d'un organosiloxane porteur d'au moins un groupe absorbant les ultraviolets dans et pour la préparation d'une composition cosmétique ou dermatologique comme agent de protection de la couleur des fibres kératiniques teintes artificiellement contre les effets néfastes des rayonnements UV, en particulier du rayonnement solaire.

D'autres objets apparaitront à la lecture de la description et des exemples qui suivent. Les organosiloxanes portant des groupements absorbant les ultraviolets, sont connus dans l'état de la technique et comportent dans leur molécule au moins une unité de formule :

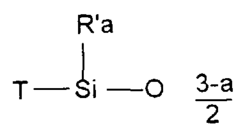

(I)

dans laquelle :

- R' désigne un groupe hydrocarboné saturé ou insaturé en $C_1$-$C_{30}$, un groupe hydrocarboné halogéné en $C_1$-$C_8$, un groupe hydrocarboné aromatique ou un groupe triméthylsiloxy ;
- a = 1 ou 2 ;
- T = -E-U où E représente un radical divalent hydrocarboné aliphatique, saturé ou insaturé, linéaire ou ramifié ayant au moins 2 atomes de carbone et renfermant éventuellement un ou plusieurs atomes d'oxygène ou bien un groupe aromatique et U représente le reste d'une molécule filtrant le rayonnement ultraviolet.

En plus des unités de formule (I), le polyorganosiloxane peut comporter des unités de formule (II) et/des unités de formule (III)

$$R'b — Si — O_{\frac{4-b}{2}} \qquad (II)$$

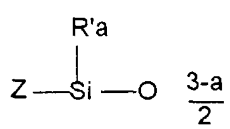

(III)

dans lesquelles :

- R' et a ont les même significations que dans la formule (I) ;
- b = 1, 2 ou 3 ;

Z = -O-U, U ayant la même signification que dans la formule (I).

A titre de groupe hydrocarboné, on peut citer les radicaux alkyle en $C_1$-$C_{30}$, alcényle en $C_2$-$C_{30}$, cycloalkyle, aromatique comme phényle ou toluyle.

A titre de groupe hydrocarboné halogéné, on peut citer le radical 3,3,3-trifluoropropyle.

Dans les organopolysiloxanes, constitués d'unités de formule (I) et éventuellement d'unités de formule (II) et/ou de formule (III), de façon préférentielle, au moins 40% en nombre des radicaux R' sont des radicaux méthyle. Le nombre total des unités (I), (II) et (III) est de préférence inférieur ou égal à 250 et varie en particulier de 2 à 50.
U représente de préférence un reste :

- benzylidène camphre éventuellement substitué sur le noyau benzénique par des radicaux hydroxyle, alkyle ou alcoxy en $C_1$-$C_8$ ;
- benzalmalonate de dialkyle en $C_1$-$C_8$ éventuellement substitué sur le noyau benzénique par des radicaux hydroxyle, alkyle ou alcoxy en $C_1$-$C_8$ ;
- 2-(2'-hydroxyphényl)benzotriazole portant éventuellement sur l'un des noyaux aromatiques des substituants alkyle en $C_1$-$C_8$ ou alcényle en $C_2$-$C_8$, halogène, alcoxy, carboxy, hydroxy, amino ou tétraalkylpipéridyle ;
- dibenzoylméthane portant éventuellement des radicaux alkyle ou alcoxy en $C_1$-$C_8$ ou hydroxy ;
- benzophénone portant éventuellement des radicaux alkyle ou alcoxy en $C_1$-$C_8$ ou hydroxy ;
- benzoate substitué par des radicaux hydroxy, alcoxy en $C_1$-$C_6$, amino ou mono- ou di($C_1$-$C_6$ alkyl)amino ;
- cinnamate portant éventuellement des radicaux hydroxy, alkyle ou alcoxy en $C_1$-$C_6$, amino ou mono- ou di($C_1$-$C_6$ alkyl)amino ;
- bis- ou tri-(phénylacrylate) éventuellement substitué par des radicaux hydroxy ou alcoxy en $C_1$-$C_4$.

Ces organopolysiloxanes porteurs de groupes absorbant les ultraviolets sont décrits notamment dans les demandes de brevet européen n° 0335777, 0305059, 0392882, 0392883, 0388218, 0350314, 0383655, 0389337, 0 709 080, 0 538 431, 0 708 108, 0711 779 ; dans la demande de brevet internationale WO 92/20690, dans les demandes de brevet français n° 2 550 787, 2 657 351, 2 642 967, 2 642 968 et dans les brevets américains USP n° 4 696 969, 4 554 369, 4 562 278, 3 513 184, 4 859 759.

Les silicones filtres benzotriazoles conformes à l'invention sont choisies parmi celles qui répondent aux formules suivantes:

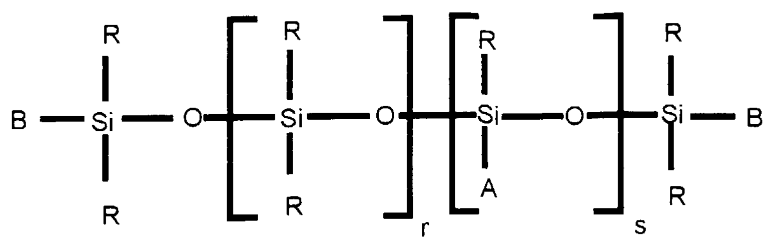

(1)

ou ou

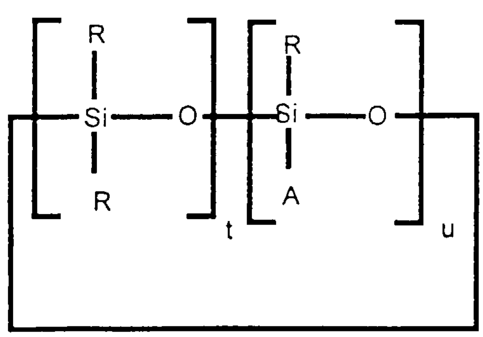

(2)

formules (1) et (2) dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

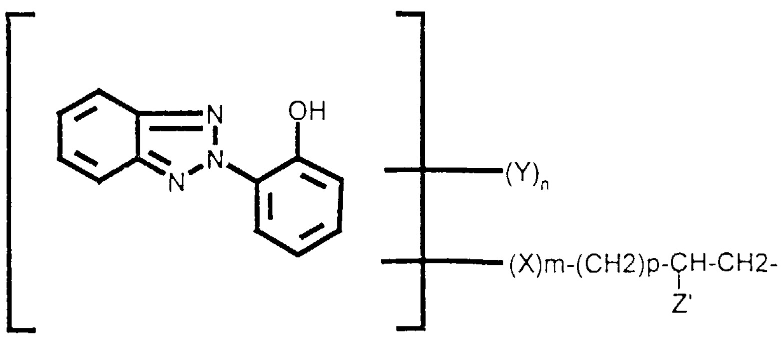

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z' représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Comme cela ressort de la formule (3) donnée ci-dessus, l'accrochage du chaînon $-(X)_m-(CH_2)_p-CH(Z')-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

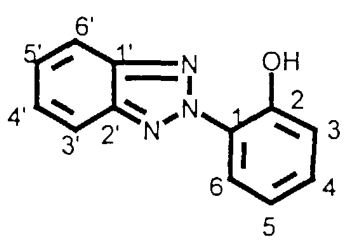

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (1) et (2) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R préférés selon l'invention

sont les radicaux méthyle, éthyle, propyle, n-butyle, n-octyle et éthyl-2 hexyle. Encore plus préférentiellement, les radicaux R sont tous des radicaux méthyle.

Ces silicones filtres sont décrites ainsi que leur procédé de synthèse dans la demande EP-A-0 392 883 et dans la demande WO 94/06404.

Parmi les composés de formules (1) ou (2) ci-dessus, on préfère mettre en oeuvre ceux répondant à la formule (1), c'est-à-dire des diorganosiloxanes à chaîne courte linéaire.

Parmi les composés de formules (1) ci-dessus, on préfère mettre en oeuvre ceux pour lesquels les radicaux B sont tous les deux des radicaux R.

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :

- B est un radical R,
- R est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, ter.-butyle ou alcoxy en $C_1$-$C_4$,
- Z' est hydrogène ou méthyle,
- m=0, ou [m=1 et X=O]
- p est égal à 1.

Une famille de composés convenant particulièrement à l'invention est celle définie par la formule générale (4) suivante :

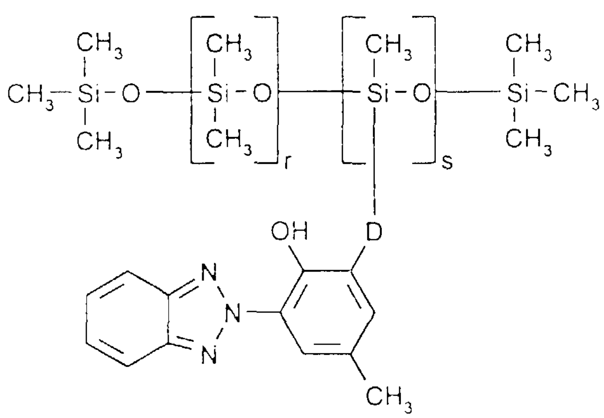

(4)

avec

$0 \leq r \leq 10$ ,

$1 \leq s \leq 10$ , et où D représente le radical divalent :

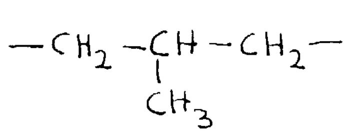

Dans une forme particulièrement préférée de réalisation de l'invention, la silicone benzotriazole est le composé (appelé composé (c) dans la suite du texte) répondant à la formule suivante :

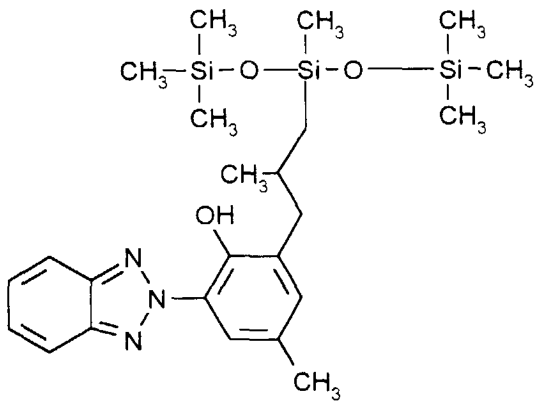

composé (c)

Les silicones benzalmalonates, conformes à l'invention comportent au moins des unités répondant à l'une des deux formules suivantes (5) et (6) :

$$O_{\frac{3-c}{2}}Si(R'')c — M — O — C_6R'''_2H_2 — CH{=}C — \left[C(O)OR''''\right]_2 \qquad (5)$$

$$O_{\frac{3-c}{2}}Si(R'')c — M' — O — C_6R'''_2H_2 — CH{=}C — \left[C(O)OR''''\right]_2 \qquad (6)$$

dans lesquelles :

- M désigne un groupe de structure :

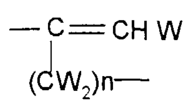

ou

$$—CW{=}CH—(CW_2)n—$$

- M' désigne un groupe de structure :

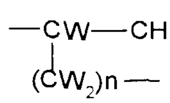

ou

$$—CW_2—CHW—(CW_2)n—$$

les autres unités de la silicone présentes étant de structure :

$$Qd - Si - O_{\frac{4-d}{2}} \qquad (7)$$

où R" désigne un radical alkyle en $C_1$-$C_8$ ou aryle ; W est un hydrogène. ou un radical alkyle en $C_1$-$C_5$ ; R''' est un hydrogène. ou un radical alkyle en $C_1$-$C_5$ ou un radical OW ; R"" désigne un groupe alkyle en $C_1$-$C_5$ ; Q désigne un hydrogène, un radical hydrocarboné monovalent en $C_1$-$C_8$ ou un groupe hydrocarboné halogéné ; c vaut 0, 1 ou 2 ; d vaut 0, 1, 2 ou 3 et n a une valeur de 1 à 6 ; sous réserve que le groupe -M-O- ou -M'-O- soit relié au noyau aromatique en position méta ou para par rapport au groupe :

$$-CH=C-\left[C(O)OR''''\right]_2$$

et que les deux groupes R''' occupent les autres positions restantes du noyau aromatique.

Ces silicones filtres sont décrites ainsi que leurs procédés de préparation dans les demandes de brevet EP-A-0 392 882, EP-0 538 431, EP-A-0 709 080 et WO92/20690.

Dans une forme particulièrement préférée de réalisation de l'invention, une famille de composés particulièrement recherchée est celle définie par les silicones benzalmalonates choisies dans le groupe constitué par:

(i) les silicones répondant à la formule (8) suivante :

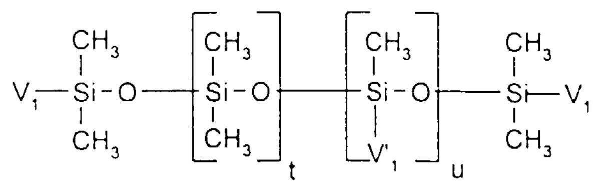

(8)

dans laquelle :

V'$_1$ désigne le groupe de structure :

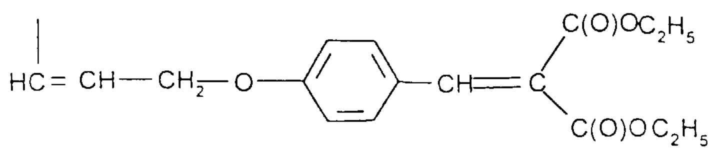

$V_1$ désigne $CH_3$ ou V'$_1$ ;

(ii) les silicones répondant à la formule (9) suivante :

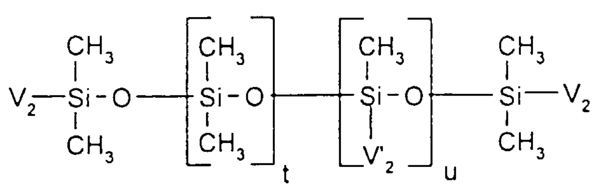

(9)

V'$_2$ désigne le groupe de structure :

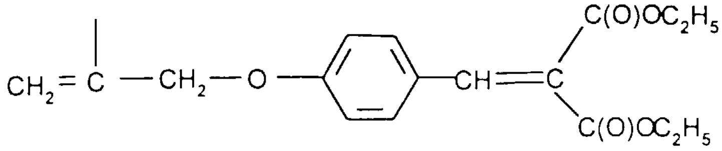

$V_2$ désigne $CH_3$ ou $V'_2$ ;

(iii) leurs mélanges ;
avec $0 \leq t \leq 100$ et $0 \leq u \leq 20$ sous réserve que :

- lorsque $V_1 = V'_1$ et/ou $V_2 = V'_2$ alors u = 0 ; et
- lorsque $V_1 = CH_3$ et/ou $V_2 = CH_3$ alors $1 \leq u \leq 20$.

Les organosiloxanes filtres de l'invention sont utilisés de préférence dans des quantités au moins égales à 0,01% en poids et généralement allant de 0,1 à 20% en poids et plus particulièrement de 0,1 à 10% en poids par rapport au poids de la composition destinée à être appliquée sur les fibres kératiniques colorées naturellement ou teintes artificiellement.

Les compositions destinées à être appliquées sur les fibres kératiniques colorées, conformément à l'invention peuvent se présenter sous forme de lotion huileuse, alcoolique ou hydroalcoolique, sous forme d'émulsion, sous forme de dispersion aqueuse ou hydroalcoolique.

Lorsque les compositions constituent des lotions huileuses, elles contiennent outre l'organosiloxane porteur d'un groupe absorbant les ultraviolets, des huiles minérales, végétales, animales ou synthétiques et plus particulièrement des isoparaffines ou des huiles de silicone à structure linéaire ou cyclique comme les polyalkyl-siloxanes, les polyarylsiloxanes, les polyalkylarylsiloxanes ou les polyorganosiloxanes modifiés par des groupements organofonctionnels non-chromophores.

Les compositions huileuses peuvent également contenir des cires, des résines ou des gommes de silicone conjointement aux huiles définies ci-dessus.

Les lotions alcooliques contiennent, outre l'organosiloxane porteur d'un groupe absorbant les ultraviolets, par exemple un alcool inférieur comportant 1 à 4 atomes de carbone et de préférence l'éthanol ou l'isopropanol ou bien d'autres alcools tels que les alkylèneglycols ou les éthers de glycols.

Lorsque les compositions de l'invention sont des émulsions, la phase grasse des émulsions est constituée, soit uniquement de l'organosiloxane filtre, soit par un mélange de celui-ci avec d'autres huiles ou cires telles que définies précédemment. L'autre phase des émulsions est constituée par un milieu aqueux.

Les émulsions non-ioniques de l'invention contiennent un émulsionnant non-ionique choisi par exemple parmi les alcools gras polyoxyéthylénés, les acides gras polyoxyéthylénés, les esters de sorbitan éventuellement polyoxyéthylénés, les alkylphénols polyoxyéthylénés ou polyglycérolés, les amides gras polyoxyéthylénés ou polyglycérolés, les alcools gras et alpha-diols polyglycérolés ; le nombre de moles d'oxyde d'éthylène variant de préférence de 2 à 50 et le nombre de groupements polyglycérolés variant de préférence de 2 à 30.

Les émulsions cationiques de l'invention contiennent un émulsionnant cationique choisi par exemple parmi les halogénures d'ammonium quaternaire tels que de dialkyl ($C_{10}$-$C_{30}$) diméthylammonium, d'alkyl ($C_{10}$-$C_{30}$) triméthylammonium, ou d'alkyl($C_{10}$-$C_{30}$) benzyl diméthylammoium et les sels d'ammoniums quaternaires polyoxyéthylénés comportant de 2 à 30 moles d'oxyde d'éthylène. On utilise plus particulièrement le chlorure de distéaryl diméthylammonium et le chlorure de béhényl triméthylammonium.

Lorsque les compositions de l'invention sont des dispersions, elles contiennent en général, de l'eau et éventuellement un alcool tel que ceux indiqués précédemment, un agent de dispersion ou un agent de mise en suspension de la silicone filtre dans l'eau. On peut citer, par exemple, comme agent de dispersion, les copolymères acrylate d'ammonium/acrylamide et les polymères d'acide acrylique réticulé.

Les compositions conformes à l'inventions peuvent également tout autre additif habituellement appliqué sur les fibres kératiniques et en particulier les cheveux tels que par exemple des colorants, des tensio-actifs, des polymères, des épaississants, des agents de conditionnement, des parfums, des conservateurs, des adoucissants ainsi que d'autres agents filtrants.

Les compositions conformes à l'invention peuvent également se présenter sous forme de spray ou être pressurisées dans des dispositifs aérosols.

Les compositions de l'invention destinées à être appliquées sur des fibres kératiniques colorées artificiellement et notamment des cheveux humains, sont des produits rincés appliqués après teinture tels que des shampooings, des après-shampooings ou bien des produits non-rincés appliqués après teinture tels que des lotions, des gels, des

mousses de coiffage, des laques aérosols ou des spray.

Les fibres kératiniques teintes artificiellement et en particulier les cheveux, sont colorés en mettant en oeuvre un agent oxydant tel que l'eau oxygénée ou l'air. On peut aussi envisager la teinture des fibres kératiniques en présence de l'organosiloxane filtre. On peut également envisager d'appliquer une composition selon l'invention à base de l'organosiloxane filtre avant la teinture des fibres kératiniques.

Les exemples qui suivent servent à illustrer l'invention sans toutefois présenter un caractère limitatif.

**EXEMPLES DE FORMULATION**

**EXEMPLE 1 Après-shampooing protecteur de la couleur des cheveux**

- Organosiloxane à fonction benzotriazole de formule (4) 1 g
- Cyclopentadiméthylsiloxane vendu sous le nom DC245 par DOW CORNING 15 g
- Cyclotétradiméthylsiloxane vendu sous le nom DC244 par DOW CORNING 15 g
- Polydiméthylsiloxane $\alpha$, $\Omega$-dihydroxyle/ silicone volatile vendu sous le nom Q2 1401 par Dow CORNING 20 g
- Ethanol 5 g
- Eau qsp 100 g

**EXEMPLE 2 Gel conditionneur après-shampooing**

- Organosiloxane à fonction benzotriazole de formule (4) 6 g
- Polydiméthylsiloxane $\alpha$, $\Omega$-dihydroxyle/silicone volatile vendu sous le nom Q2 1401 par Dow CORNING 20 g
- Copolymère réticulé acrylamide/acide 2-acrylamido-2-méthylpropane sulfonique vendu sous le nom SEPIGEL 305 par SEPPIC 1 g MA
- Eau qsp 100 g

Ce gel permet de réduire sensiblement la dégradation de la couleur des cheveux liée à l'action des agents extérieurs notamment de la lumière.

**EXEMPLE 3 Gel conditionneur après-shampooing**

- Organosiloxane à fonction benzotriazole de formule (4) 6 g
- Polydiméthylsiloxane vendu sous le nom SILBIONE V 500000 par RHONE POULENC 10 g
- Copolymère réticulé acrylamide/acide 2-acrylamido-2-méthyl propane sulfonique vendu sous le nom SEPIGEL 305 par SEPPIC 1 g MA
- Eau qsp 100 g

Ce gel permet de réduire sensiblement la dégradation de la couleur des cheveux liée à l'action des agents extérieurs notamment de la lumière.

**EXEMPLE 4 Shampooing protecteur de la couleur des cheveux**

- Organosiloxane à fonction benzalmalonate de formule (8) ou (9) 1 g
- Lauryl éther sulfate de sodium à 2,2 moles d'oxyde d'éthylène 15 g M.A.
- Cocoylbétaïne 2,4 g M.A.
- Distéarate d'éthylène glycol 2,5 g
- Monoisopropanolamide d'acide de coprah 2,5 g
- Eau qsp 100 g

Ce shampooing présente de bonnes propriétés de lavage et permet après applications et rinçage de réduire

sensiblement la dégradation de la couleur des cheveux liée à l'action des agents extérieurs notamment de la lumière.

**EXEMPLE COMPARATIF**

On effectue sur une première série de mèches de cheveux de 1,2 g légèrement sensibilisés, une teinture par oxydation conduisant à une nuance «blond foncé cendré».

On effectue sur une seconde série de mèches de cheveux de 1,2 g légèrement sensibilisés, une teinture par oxydation conduisant à une nuance «blond foncé doré acajou».

Les mèches ainsi teintes sont ensuite traitées par la composition A, B ou C suivante selon le mode opératoire indiquée ci-après :

**COMPOSITION A (invention)** :

- Organosiloxane à fonction benzotriazole de formule (4) 1 % en poids
- Ethanol absolu qsp 100 % en poids

**COMPOSITION B (invention)** :

- Organosiloxane à fonction benzalmalonate de formule (8) ou (9) 1 % en poids
- Ethanol absolu qsp 100 % en poids

**COMPOSITION C (témoin) :**

- Ethanol absolu 100 % en poids

***Mode opératoire* :**

Chaque mèche colorée est immergée pendant 10 secondes dans 400 ml d'eau permutée. Elle est ensuite essorée entre 2 feuilles de papier (2 fois). Chaque mèche colorée est ensuite traitée par 1 ml de composition A, B ou C à tester pendant 10 mn. Après essorage entre deux doigts, les mèches sont séchées au casque pendant 30 mn à 65 °C.

Les mèches ainsi traitées subissent ensuite un test de résistance à la lumière (Xenotest).

Pour ce faire, les mèches de cheveux teintes ont été fixées sur un support (carton ou plastique). Ces supports ont été disposés sur des porte-échantillons qui tournent autour d'une lampe Xénon pendant une durée de 60 heures sous un taux d'humidité relative de 25 ± 5 % et à une température de 42,5 ± 2,5°C.

Les couleurs des mèches ont été évaluées dans le système L a b, au moyen d'un colorimètre CM 2002 MINOLTA.

On mesure dans ce système pour chaque mèche :

- la couleur de la mèche obtenue après traitement par la composition A, B ou C et avant le test de résistance à la lumière et
- la couleur de la mèche obtenue après le test de résistance à la lumière.

Selon ce système, L indique la clarté. Plus la valeur de L est élevée, plus la nuance est claire. Inversement, Plus la valeur de L est faible, plus la nuance est sombre.

La nuance et la saturation sont exprimées par a et b.

a et b indiquent deux axes de nuances, a indiquant l'axe des rouges/verts et b l'axe des jaunes/bleus. Les valeurs proches de zéro pour a ou b correspondent aux nuances grises.

La différence de couleur de chaque mèche avant et après le test de résistance à la lumière reflète la dégradation de la coloration due à l'action de la lumière et a été calculée en appliquant l'équation suivante :

$$\Delta E = \sqrt{(L-L_o)^2 + (a-a_o)^2 + (b-b_o)^2}$$

Dans cette formule, Δ**E** représente la différence de couleur entre deux mèches, L, a et b représentent respectivement la clarté, la nuance et la saturation après le test et $L_0$, $a_0$ et $b_0$ représentent respectivement la clarté, la nuance et la saturation avant le test.

Les résultats des tests sont indiqués dans les tableau I et II ci-dessous :

Tableau I

| Composition | Couleur avant le test sur cheveux teints en blond foncé cendré | | | Couleur après le test sur cheveux teints en blond foncé cendré | | | Dégradation de la couleur ΔE. sur cheveux teints en blond foncé cendré |
|---|---|---|---|---|---|---|---|
| | L | a | b | L | a | b | |
| A(invention) | 22,16 | 2,39 | 4 | 28,54 | 4,06 | 10,84 | 9,50 |
| B(invention) | 22,6 | 2,35 | 3,91 | 27,91 | 3,77 | 11,19 | 9,12 |
| C (témoin) | 22,58 | 2,23 | 3,6 | 30,07 | 4,52 | 13,07 | 12,29 |

On constate que les compositions A et B selon l'invention contenant une silicone filtre réduisent respectivement de 23 et de 26 % la dégradation de la couleur des mèches teintes artificiellement, après 60 heures d'exposition à la lumière, par rapport à la composition C dépourvue de silicone filtre.

Tableau II

| Composition | Couleur avant le test sur cheveux teints en blond foncé doré acajou | | | Couleur après le test sur cheveux teints en blond foncé doré acajou | | | Dégradation de la couleur ΔE. sur cheveux teints en blond foncé doré acajou |
|---|---|---|---|---|---|---|---|
| | L | a | b | L | a | b | |
| A(invention) | 33,79 | 10,04 | 16,55 | 43,17 | 8,1 | 21,81 | 10,93 |
| B(invention) | 33,75 | 10,21 | 16,95 | 41,67 | 8,26 | 21,94 | 9,56 |
| C (témoin) | 34,59 | 10 | 16,82 | 45,96 | 7,98 | 23,54 | 13,36 |

On constate que les compositions A et B selon l'invention contenant une silicone filtre réduisent respectivement de 18 et de 28 % la dégradation de la couleur des mèches teintes artificiellement, après 60 heures d'exposition à la lumière, par rapport à la composition C dépourvue de silicone filtre.

## Revendications

**1.** Utilisation d'une silicone filtrant les rayonnements UV choisie dans le groupe constitué par :

(i) les silicones benzotriazoles répondant à l'une des formules suivantes:

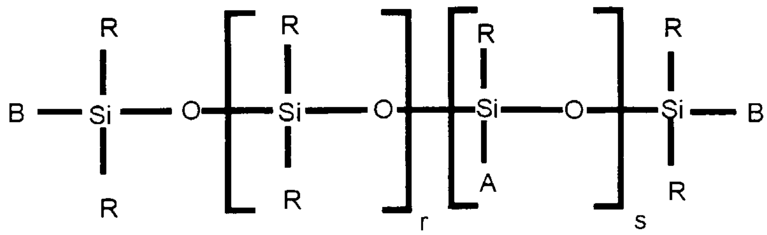

(1)

ou

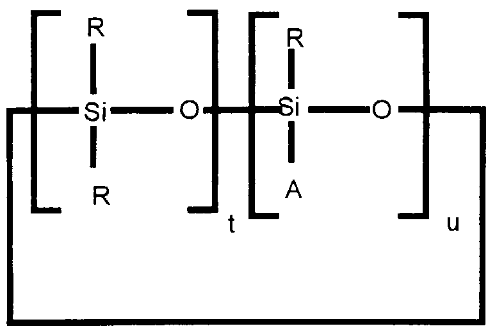

(2)

formules (1) et (2) dans lesquelles :

- R, identiques ou différents, sont choisis parmi les radicaux alkyles en $C_1$-$C_{10}$, phényle et trifluoro-3,3,3 propyle, au moins 80% en nombre des radicaux R étant méthyle,
- B, identiques ou différents sont choisis parmi les radicaux R et le radical A,
- r est un nombre entier compris entre 0 et 50 inclusivement, et s est un nombre entier compris entre 0 et 20 inclusivement, et si s = 0, au moins l'un des deux symboles B désigne A,
- u est un nombre entier compris entre 1 et 6 inclusivement, et t est un nombre entier compris entre 0 et 10 inclusivement, étant entendu que t + u est égal ou supérieur à 3,
- et le symbole A désigne un radical monovalent lié directement à un atome de silicium, et qui répond à la formule (3) suivante :

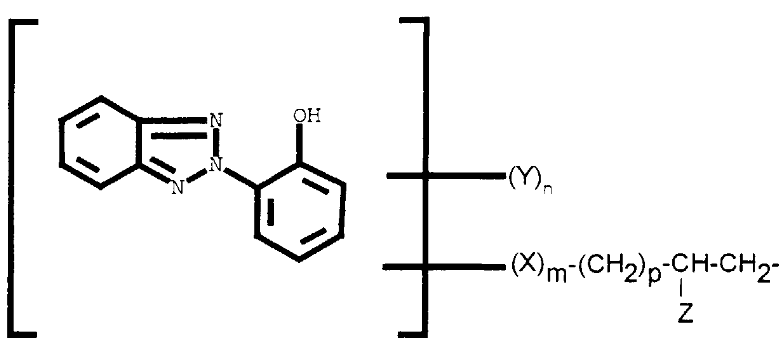

(3)

formule (3) dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,

- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

(ii) les silicones benzalmalonates comportant au moins des unités répondant à l'une des deux formules suivantes (5) et (6) :

$$O_{\frac{3-c}{2}}Si(R'')c—M—O—C_6R'''_2H_2—CH{=}C—\left[C(O)OR''''\right]_2 \quad (5)$$

$$O_{\frac{3-c}{2}}Si(R'')c—M'—O—C_6R'''_2H_2—CH{=}C—\left[C(O)OR''''\right]_2 \quad (6)$$

dans lesquelles :

- M désigne un groupe de structure :

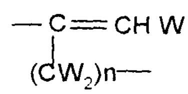

ou

$$—CW—CH—(CW_2)n—$$

- M' désigne un groupe de structure :

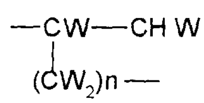

ou

$$—CW_2—CHW—(CW_2)n—$$

les autres unités de la silicone présentes étant de structure :

$$Qd—Si—O_{\frac{4-d}{2}} \quad (7)$$

où R'' désigne un radical alkyle en $C_1$-$C_8$ ou aryle ; W est un hydrogène ou un radical alkyle en $C_1$-$C_5$ ; R''' est un hydrogène.ou un radical alkyle en $C_1$-$C_5$ ou un radical OW ; R'''' désigne un groupe alkyle en $C_1$-$C_5$ ; Q désigne un hydrogène, un radical hydropcarboné monovalent en $C_1$-$C_8$ ou un groupe hydrocarboné halogéné ; c vaut 0, 1 ou 2 ; d vaut 0, 1, 2 ou 3 et n a une valeur de 1 à 6 ; sous réserve que le groupe -M-O-ou -M'-O- soit relié au noyau aromatique en position méta ou para par rapport au groupe

$$-CH=C-[C(O)OR'''']_2$$

et que les deux groupes R''' occupent les autres positions restantes du noyau aromatique ; pour la préparation d'une composition cosmétique ou dermatologique comme agent de protection de la couleur de fibres kératiniques contre les effets néfastes des rayonnements UV ; lesdites fibres étant teintes selon un procédé de coloration utilisant des précurseurs de colorant d'oxydation et un agent oxydant.

**2.** Utilisation selon la revendication 1, caractérisée par le fait que les fibres kératiniques sont des cheveux humains.

**3.** Utilisation selon la revendication 1, caractérisée par le fait que la silicone benzotriazole est choisie parmi les composés de formule générale (1) pour lesquels les radicaux B sont choisis parmi les radicaux R.

**4.** Utilisation selon la revendication 1 ou 3, caractérisée par le fait que la silicone benzotriazole est choisie au sein de celles présentant au moins l'une des caractéristiques suivantes :

- R est méthyle,
- r est compris entre 0 et 15 inclusivement ; s est compris entre 1 et 10 inclusivement,
- n est égal à 1, et Y est alors choisi parmi méthyle, ter.-butyle ou alcoxy en $C_1$-$C_4$,
- Z est hydrogène ou méthyle,
- m=0, ou [m=1 et X=O]
- p est égal à 1.

**5.** Utilisation selon la revendication 4, caractérisée par le fait que ladite silicone benzotriazole présente l'ensemble desdites caractéristiques.

**6.** Utilisation selon la revendication 1, caractérisée par le fait que la silicone benzotriazole est choisie parmi les composés de formule générale (4) :

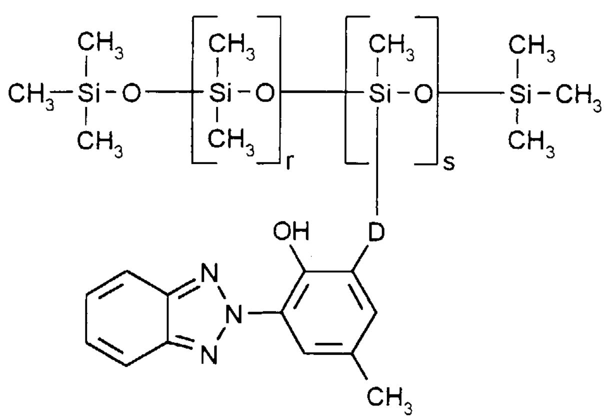

(4)

avec

$0 \leq r \leq 10$,

$1 \leq s \leq 10$, et où D représente le radical divalent :

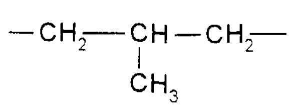

**7.** Utilisation selon la revendication 6, caractérisée par le fait que la silicone benzotriazole répond à la formule

suivante :

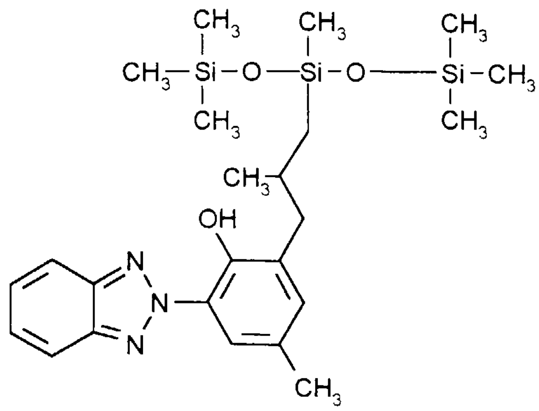

**8.** Utilisation selon la revendication 1, selon laquelle la silicone benzalmalonate est choisie dans le groupe constitué par :

(i) les silicones répondant à la formule (8) suivante :

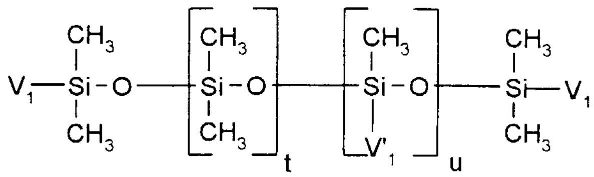

(8)

dans laquelle :
$V'_1$ désigne le groupe de structure :

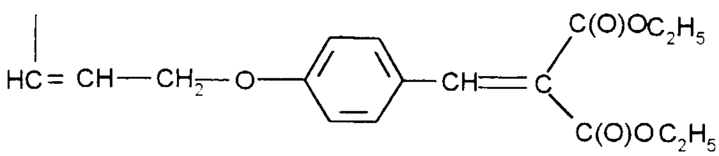

$V_1$ désigne $CH_3$ ou $V'_1$ ;

(ii) les silicones répondant à la formule (9) suivante :

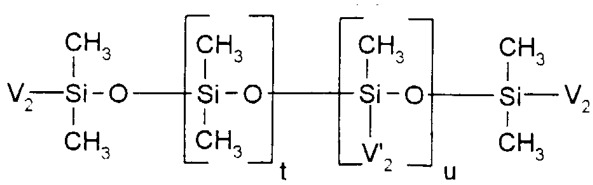

(9)

où $V'_2$ désigne le groupe de structure :

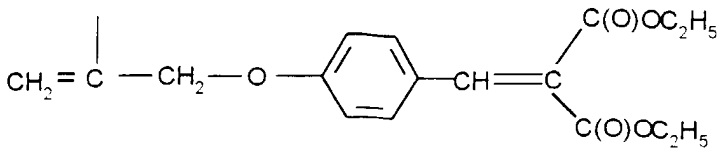

$V_2$ désigne $CH_3$ ou $V'_2$ ;

(iii) leurs mélanges ;
avec $0 \leq t \leq 100$ et $0 \leq u \leq 20$ sous réserve que :

- lorsque $V_1 = V'_1$ et/ou $V_2 = V'_2$ alors u = 0 ; et
- lorsque $V_1 = CH_3$ et/ou $V_2 = CH_3$ alors $1 \leq u \leq 20$.

**9.** Utilisation selon l'une quelconque des revendications 1 à 8, selon laquelle la silicone filtre est utilisée dans des quantités au moins égales à 0,01% en poids par rapport au poids de la composition destinée à être appliquée sur les fibres kératiniques.

**10.** Utilisation selon la revendication 9, selon laquelle la silicone filtre est utilisée dans des quantités allant de 0,1 à 20% en poids par rapport au poids de la composition destinée à être appliquée sur les fibres kératiniques.

**11.** Utilisation selon l'une quelconque des revendications 1 à 10, selon laquelle la composition destinée à être appliquée sur les fibres kératiniques se présente sous forme de lotion huileuse, de lotion alcoolique, de lotion hydroalcoolique, d'émulsion, de dispersion aqueuse ou hydroalcoolique.

**12.** Utilisation selon l'une quelconque des revendications 1 à 11, pour la préparation d'un produit capillaire à rincer après application.

**13.** Utilisation selon l'une quelconque des revendications 1 à 11, pour la préparation d'un produit capillaire non rincé après application.

**14.** Utilisation selon l'une quelconque des revendications 1 à 11, pour la préparation d'un produit capillaire appliqué avant, pendant ou après teinture des cheveux.

**Patentansprüche**

**1.** Verwendung eines Silicons, das die UV-Strahlung filtert und ausgewählt ist unter:

(i) den Benzotriazolsiliconen, die einer der folgenden Formeln entsprechen:

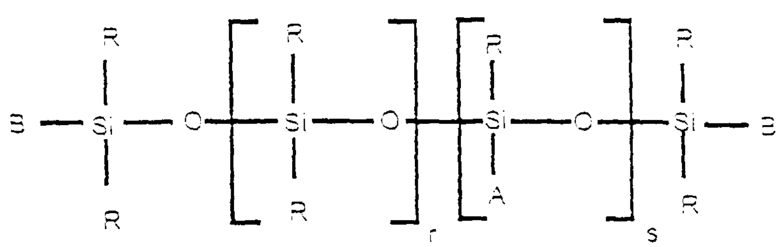

(1)

oder

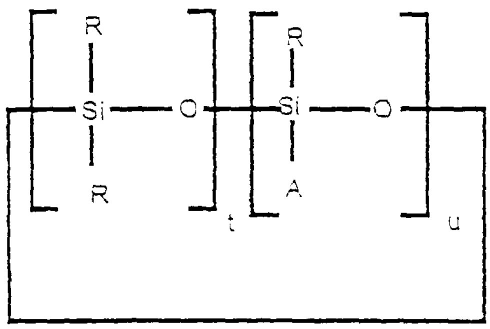

(2),

worin:

- die Gruppen R, die identisch oder voneinander verschieden sind, unter den $C_{1-10}$-Alkylgruppen, Phenyl und 3,3,3-Trifluorpropyl ausgewählt sind, wobei mindestens 80 % der Gruppen R Methyl bedeuten,
- die Gruppen B, die identisch oder voneinander verschieden sind, unter den Gruppen R und der Gruppe A ausgewählt sind,
- r 0 oder eine ganze Zahl im Bereich von 1 bis 50 und s 0 oder eine ganze Zahl im Bereich von 1 bis 20 bedeutet, und mindestens eine der beiden Gruppen B A bedeutet, wenn s 0 ist,
- u eine ganze Zahl im Bereich von 1 bis 6 und t 0 oder eine ganze Zahl im Bereich von 1 bis 10 ist, mit der Maßgabe, daß t + u 3 bedeutet oder darüber liegt, und
- die Gruppe A eine einwertige Gruppe bedeutet, die direkt an ein Siliciumatom gebunden ist und der folgenden Formel (3) entspricht:

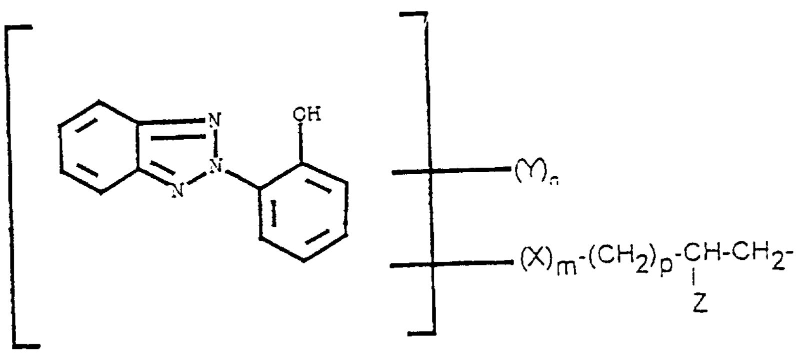

(3),

worin:

- die Gruppen Y, die identisch oder voneinander verschieden sind, unter den $C_{1-8}$-Alkylgruppen, Halogenen und $C_{1-4}$-Alkoxygruppen ausgewählt sind, wobei im letzten Fall zwei angrenzende Gruppen Y an dem gleichen aromatischen Ring auch gemeinsam eine Alkylidendioxygruppe bilden können, worin die Alkylidengruppe 1 bis 2 Kohlenstoffatome aufweist,
- X O oder NH bedeutet,
- Z' Wasserstoff oder eine $C_{1-4}$-Alkylgruppe bedeutet,
- n 0 oder eine ganze Zahl im Bereich von 1 bis 3 ist,
- m 0 oder 1 ist, und
- p eine ganze Zahl im Bereich von 1 bis 10 bedeutet; und

(ii) den Benzalmalonatsiliconen, die zumindest Einheiten enthalten, die einer der beiden folgenden Formeln (5) und (6) entsprechen:

$$O_{\frac{3-c}{2}}Si(R'')_c-M-O-C_6R'''_2H_2-CH=C-[C(O)OR'''']_2 \qquad (5)$$

$$O_{\frac{3-c}{2}}Si(R'')_c-M'-O-C_6R'''_2H_2-CH=C-[C(O)OR'''']_2 \qquad (6),$$

worin bedeuten:

- M eine Gruppe der Formel:

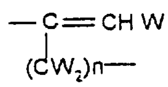

oder

$$-CW=CH-(CW_2)n-$$

- M' eine Gruppe der Formel:

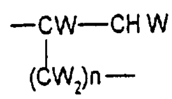

oder

$$-CW_2-CHW-(CW_2)n-$$

wobei die anderen Einheiten des Silicons die folgende Struktur aufweisen:

$$Qd-Si-O_{\frac{4-d}{2}} \qquad (7),$$

worin bedeuten: R'' eine $C_{1-8}$-Alkylgruppe oder Aryl; W Wasserstoff oder eine $C_{1-5}$-Alkylgruppe; R''' Wasserstoff, eine $C_{1-5}$-Alkylgruppe oder eine Gruppe OW; R'''' eine $C_{1-5}$-Alkylgruppe; Q Wasserstoff, eine einwertige Kohlenwasserstoffgruppe mit 1 bis 8 Kohlenstoffatomen oder eine halogenierte Kohlenwasserstoffgruppe; c 0, 1 oder 2; d 0, 1, 2 oder 3 und n eine Zahl von 1 bis 6; mit der Maßgabe, daß die Gruppe -M-O- oder -M'-O- in bezug auf die Gruppe:

$$-CH=C-[C(O)OR'''']_2$$

in meta- oder para-Stellung an den aromatischen Ring gebunden ist und die beiden Gruppen R''' die übrigen Stellungen des aromatischen Rings einnehmen,
zur Herstellung einer kosmetischen oder dermatologischen Zusammensetzung als Mittel zum Schutz der Farbe von Keratinfasern gegen die schädlichen Wirkungen der UV-Strahlung, wobei die Fasern nach einem Verfahren zum Färben unter Verwendung von Farbstoffvorprodukten von Oxidationsfarbstoffen und einem Oxidationsmittel gefärbt wurden.

**2.** Verwendung nach Anspruch 1, dadurch gekennzeichnet, daß es sich bei den Keratinfasern um menschliches Haar handelt.

**3.** Verwendung nach Anspruch 1, dadurch gekennzeichnet, daß das Benzotriazol unter den Verbindungen der allgemeinen Formel (1) ausgewählt ist, worin die Gruppen B unter den Gruppen R ausgewählt sind.

**4.** Verwendung nach Anspruch 1 oder 3, dadurch gekennzeichnet, daß das Benzotriazol unter den Verbindungen ausgewählt ist, die mindestens eine der folgenden Eigenschaften aufweisen:

- R bedeutet Methyl,
- r liegt im Bereich von 0 bis 15; s liegt im Bereich von 1 bis 10,
- n ist 1, und Y ist unter Methyl, *tert*.-Butyl oder $C_{1-4}$-Alkoxy ausgewählt,
- Z bedeutet Wasserstoff oder Methyl,
- m = 0, oder [m = 1 und X = O], und
- p bedeutet 1.

**5.** Verwendung nach Anspruch 4, dadurch gekennzeichnet, daß das Benzotriazol alle diese Eigenschaften aufweist.

**6.** Verwendung nach Anspruch 1, dadurch gekennzeichnet, daß das Benzotriazol unter den Verbindungen der allgemeinen Formel (4) ausgewählt ist:

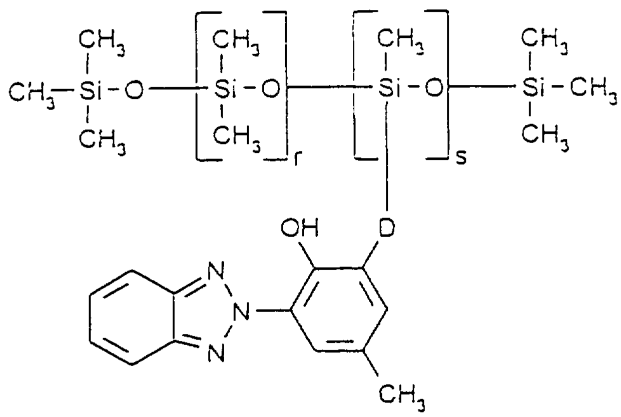

(4)

worin bedeuten:

$0 \leq r \leq 10$,

$1 \leq s \leq 10$, und D die zweiwertige Gruppe:

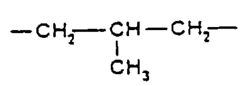

**7.** Verwendung nach Anspruch 6, dadurch gekennzeichnet, daß das Benzotrizol der folgenden Formel entspricht:

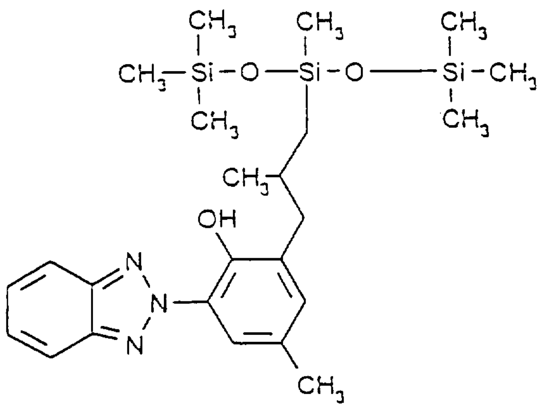

**8.** Verwendung nach Anspruch 1, dadurch gekennzeichnet, daß das Benzalmalonatsilicon ausgewählt ist unter:

(i) Siliconen, die der folgenden Formel (8) entsprechen:

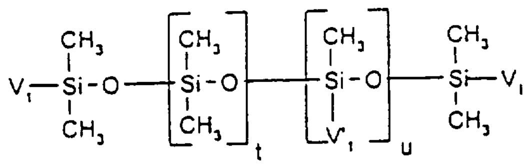

(8)

worin bedeuten:
$V'_1$ die folgende Gruppe:

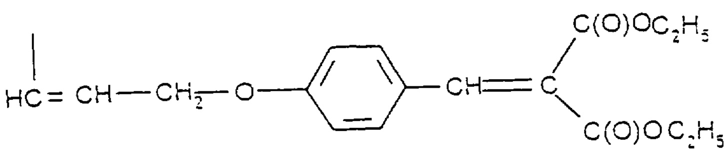

und $V_1$ $CH_3$ oder $V'_1$ ;

(ii) Siliconen, die der folgenden Formel (9) entsprechen:

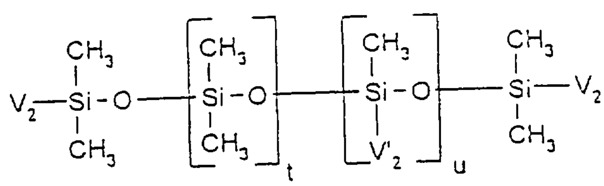

(9),

wobei $V'_2$ die folgende Gruppe bedeutet:

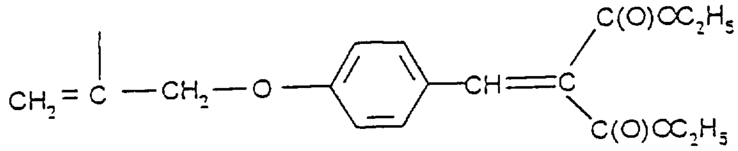

und $V_2$ $CH_3$ oder $V'_2$ bedeutet;

(iii) deren Gemischen;
mit $0 \leq t \leq 100$ und $0 \leq u \leq 20$; mit der Maßgabe, daß:

- $u = 0$, wenn $V_1 = V'_1$ und/oder $V_2 = V'_2$; und
- $1 \leq u \leq 20$, wenn $V_1 = CH_3$ und/oder $V_2 = CH_3$.

**9.** Verwendung nach einem der Ansprüche 1 bis 8, wobei das Siliconfilter in Mengenanteilen von mindestens 0,01 Gew.-%, bezogen auf das Gesamtgewicht der Zusammensetzung, die auf die Keratinfasern aufgetragen werden soll, verwendet wird.

**10.** Verwendung nach Anspruch 9, wobei das Siliconfilter in Mengenanteilen von 0,1 bis 20 Gew.-%, bezogen auf das Gesamtgewicht der Zusammensetzung, die auf die Keratinfasern aufgetragen werden soll, verwendet wird.

**11.** Verwendung nach einem der Ansprüche 1 bis 10, wobei die Zusammensetzung, die auf die Keratinfasern aufgetragen werden soll, als ölige Lotion, alkoholische Lotion, wässrig-alkoholische Lotion, Emulsion, wäßrige Dispersion oder wässrig-alkoholische Dispersion vorliegt.

**12.** Verwendung nach einem der Ansprüche 1 bis 11 zur Herstellung eines Produktes für das Haar, das nach dem Auftragen ausgespült wird.

**13.** Verwendung nach einem der Ansprüche 1 bis 11 zur Herstellung eines Produktes für das Haar, das nach dem Auftragen nicht ausgespült wird.

**14.** Verwendung nach einem der Ansprüche 1 bis 11 zur Herstellung eines Produktes für das Haar, das vor, während oder nach dem Färben der Haare aufgetragen wird.

## Claims

**1.** Use of a silicone for screening out U.V. radiation, chosen from the group consisting of:

(i) benzotriazole silicones corresponding to one of the following formulae:

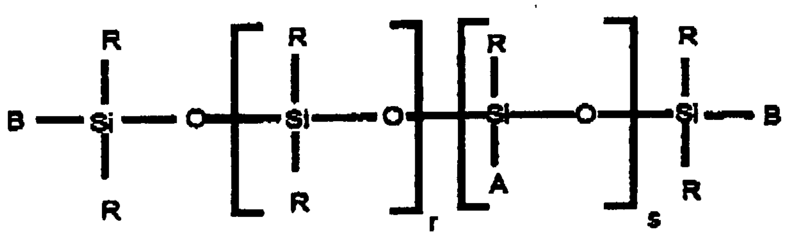

(1)

or

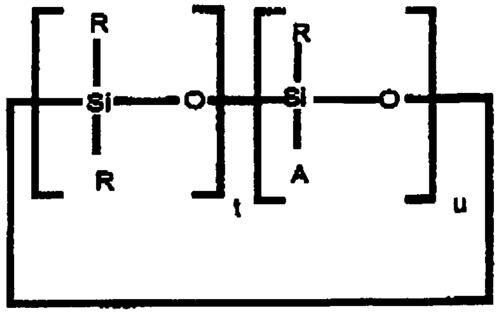

(2)

in which formulae (1) and (2):

- R, which may be identical or different, are chosen from $C_1$-$C_{10}$ alkyl, phenyl and 3,3,3-trifluoropropyl radicals, at least 80%, in numerical terms, of the radicals R being methyl,
- B, which may be identical or different, are chosen from radicals R and the radical A,
- r is an integer between 0 and 50 inclusive, and s is an integer between 0 and 20 inclusive, and if s = 0, at least one of the two symbols B denotes A,
- u is an integer between 1 and 6 inclusive, and t is an integer between 0 and 10 inclusive, it being understood that t + u is equal to or greater than 3,
- and the symbol A denotes a monovalent radical linked directly to a silicon atom and corresponding to formula (3) below:

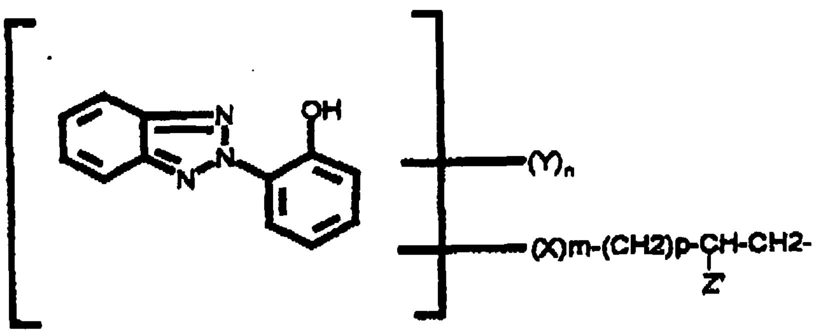

(3)

in which formula (3)

- Y, which may be identical or different, are chosen from $C_1$-$C_8$ alkyl radicals, halogens and $C_1$-$C_4$ alkoxy radicals, it being understood that, in the latter case, two adjacent Y groups of the same aromatic ring may together form an alkylidenedioxy group in which the alkylidene group contains from 1 to 2 carbon atoms,
- X represents O or NH,
- Z represents hydrogen or a $C_1$-$C_4$ alkyl radical,
- n is an integer between 0 and 3 inclusive,
- m is 0 or 1,
- p represents an integer between 1 and 10 inclusive.

(ii) benzalmalonate silicones containing at least some units corresponding to one of the two formulae (5) and (6) below:

$$O_{\frac{3-c}{2}}Si(R'')_c - M - O - C_6R'''_2H_2 - CH{=}C - [C(O)OR'''']_2 \quad (5)$$

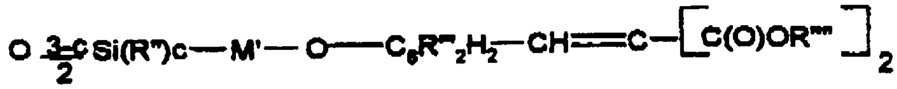 (6)

in which:

- M denotes a group having the structure:

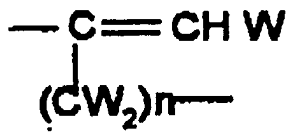

or

$$—CW=CH—(CW_2)n—$$

- M' denotes a group having the structure:

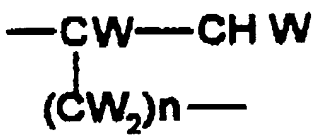

or

$$—CW_2—CHW—(CW_2)n—$$

the other units of the silicone which are present having the structure:

$$Qd—Si—O_{\frac{4-d}{2}} \quad (7)$$

where R" denotes an aryl or $C_1$-$C_8$ alkyl radical; W is a hydrogen or a $C_1$-$C_5$ alkyl radical; R"' is a hydrogen or a $C_1$-$C_5$ alkyl radical or a radical OW; R"" denotes a $C_1$-$C_5$ alkyl group; Q denotes a hydrogen, a monovalent $C_1$-$C_8$ hydrocarbon radical or a halogenated hydrocarbon group; c is equal to 0, 1 or 2; d is equal to 0, 1, 2 or 3 and n has a value from 1 to 6; with the proviso that the group -M-O- or -M'-O- is connected to the aromatic ring in a meta or para position relative to the group:

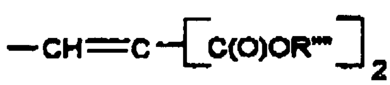

and with the proviso that the two groups R"' occupy the other remaining positions on the aromatic ring; for the preparation of a cosmetic or dermatological composition as an agent for protecting the colour of keratin fibres against the harmful effects of UV radiation; the said fibres being dyed according to a dyeing process using oxidation dye precursors and an oxidizing agent.

**2.** Use according to Claim 1, characterized in that the keratin fibres are human hair.

**3.** Use according to Claim 1, characterized in that the benzotriazole silicone is chosen from the compounds of general formula (1) in which the radicals B are chosen from the radicals R.

**4.** Use according to Claim 1 or 3, characterized in that the benzotriazole silicone is chosen from those having at least one of the following characteristics:

- R is methyl,
- r is between 0 and 15 inclusive; s is between 1 and 10 inclusive,
- n is equal to 1, and Y is then chosen from methyl, tert-butyl and $C_1$-$C_4$ alkoxy,
- Z is hydrogen or methyl,
- m=0 or [m=1 and X=O],
- p is equal to 1.

**5.** Use according to Claim 4, characterized in that the said benzotriazole silicone has all of the said characteristics.

**6.** Use according to Claim 1, characterized in that the benzotriazole silicone is chosen from the compounds of general formula (4):

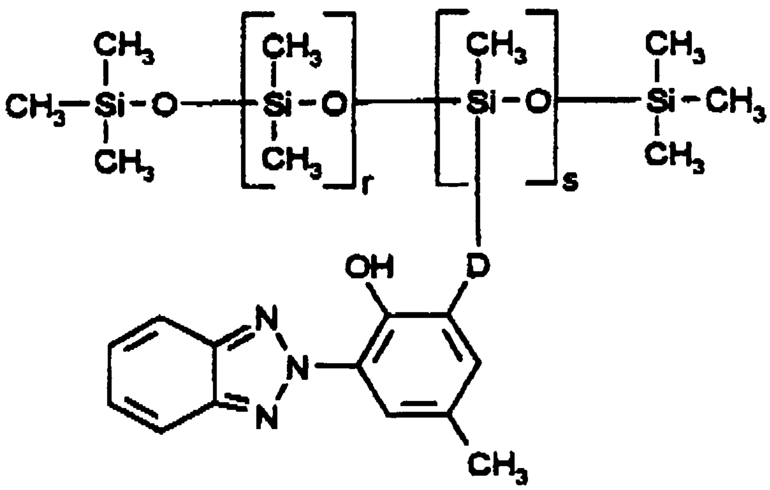

(4)

with

$0 \leq r \leq 10$,

$1 \leq s \leq 10$, and where D represents the divalent radical:

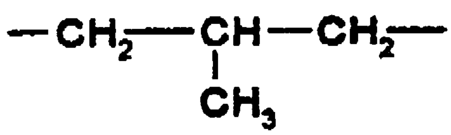

**7.** Use according to Claim 6, characterized in that the benzotriazole silicone corresponds to the following formula:

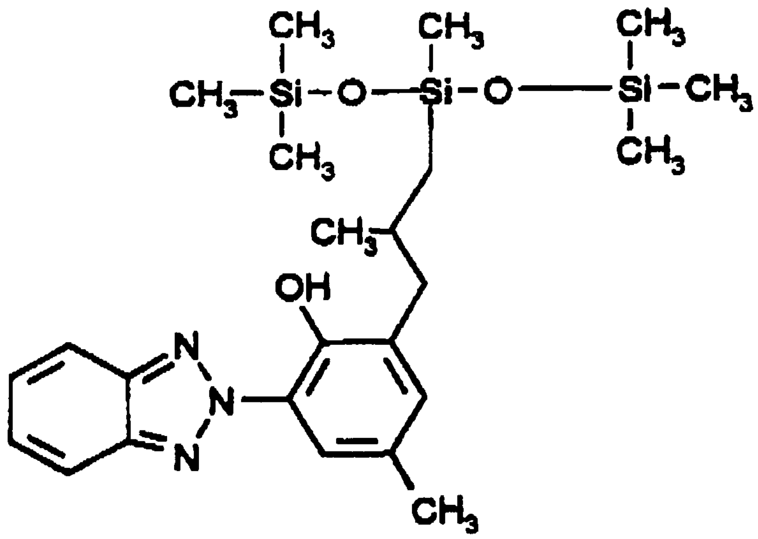

**8.** Use according to Claim 1, according to which the benzalmalonate silicone is chosen from the group consisting of:

(i) silicones corresponding to formula (8) below:

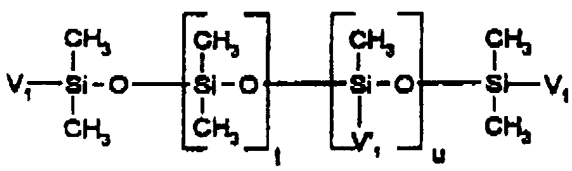

(8)

in which:
$V'_1$ denotes the group having the structure:

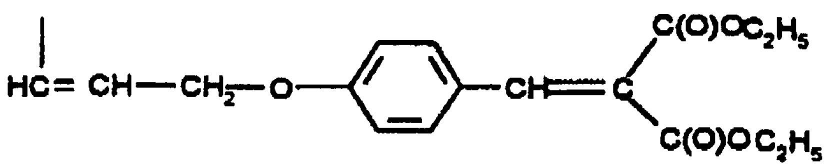

$V_1$ denotes $CH_3$ or $V'_1$;
(ii) silicones corresponding to formula (9) below:

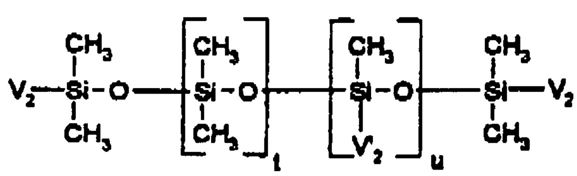

(9)

where $V'_2$ denotes the group having the structure:

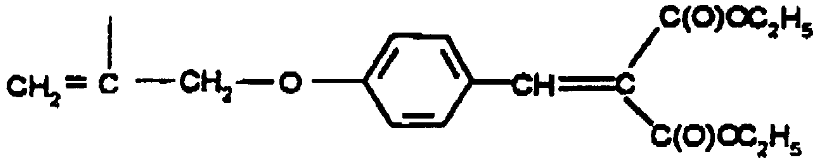

$V_2$ denotes $CH_3$ or $V'_2$;
(iii) mixtures thereof;
with $0 \leq t \leq 100$ and $0 \leq u \leq 20$, with the proviso that;

- when $V_1 = V'_1$ and/or $V_2 = V'_2$ then $u = 0$; and
- when $V_1 = CH_3$ and/or $V_2 = CH_3$ then $1 \leq u \leq 20$.

**9.** Use according to any one of Claims 1 to 8, according to which the screening silicone is used in amounts at least equal to 0.01% by weight relative to the weight of the composition intended to be applied to the keratin fibres.

**10.** Use according to Claim 9, according to which the screening silicone is used in amounts ranging from 0.1% to 20% by weight relative to the weight of the composition intended to be applied to the keratin fibres.

**11.** Use according to any one of Claims 1 to 10, according to which the composition intended to be applied to the keratin fibres is in the form of an oily lotion, an alcoholic lotion, an aqueous-alcoholic lotion, an emulsion or an aqueous or aqueous-alcoholic dispersion.

**12.** Use according to any one of Claims 1 to 11 for the preparation of a hair product to be rinsed out after application.

**13.** Use according to any one of Claims 1 to 11 for the preparation of a hair product which is not rinsed out after application.

**14.** Use according to any one of Claims 1 to 11 for the preparation of a hair product which is applied before, during or after dyeing the hair.